# EUROPEAN PATENT SPECIFICATION

(11) **EP 2 750 759 B1**
(45) Date of publication and mention of the grant of the patent: **21.12.2016**
(21) Application number: 12756585.1
(22) Date of filing: 20.08.2012
(51) Int. Cl.: A61N 1/372, A61N 1/362

(54) **ALGORITHM FOR NARRATIVE GENERATION**
ALGORITHMUS ZUR ERZEUGUNG EINER ERZÄHLUNG
ALGORITHME POUR LA GÉNÉRATION DE TEXTE NARRATIF

(30) Priority: 29.08.2011 US 201161528372 P
(43) Date of publication of application: 09.07.2014
(73) Proprietor: Cardiac Pacemakers, Inc., St. Paul, MN 55112-5798 (US)
(72) Inventor: EBERLE, LeAnne M., Mahtomedi, Minnesota 55115 (US); PERSCHBACHER, David L., Coon Rapids, Minnesota 55448 (US); WONG, Chen Yee, Shoreview, Minnesota 55126 (US); SHAO, Weiguang, Woodbury, Minnesota 55125 (US)
(74) Representative: Peterreins Schley
(86) International application number: PCT/US2012/051555
(87) International publication number: WO 2013/032760

(56) References cited:
- WO-A1-97/26043
- WO-A1-97/32272
- US-A1- 2005 273 362
- US-A1- 2007 169 021
- US-A1- 2008 270 036

## Description

The present description generally relates to narrative text generation in ambulatory medical devices. The invention is set out in the appended claims.

### BACKGROUND

Ambulatory medical devices include devices designed to be implanted into a patient. Some examples of these implantable medical devices (IMDs) include cardiac function management (CFM) devices such as implantable pacemakers, implantable cardioverter defibrillators (ICDs), cardiac resynchronization therapy devices (CRTs), and devices that include a combination of such capabilities. The devices can be used to treat patients or subjects using electrical or other therapy or to aid a physician or caregiver in patient diagnosis through internal monitoring of a patient's condition. The devices may include one or more electrodes in communication with one or more sense amplifiers to monitor electrical heart activity within a patient, and often include one or more sensors to monitor one or more other internal patient parameters. Other examples of IMDs include implantable diagnostic devices, implantable drug delivery systems, or implantable devices with neural stimulation capability.

Ambulatory medical devices also include wearable medical devices (WMDs) such as wearable cardioverter defibrillators (WCDs). WCDs are monitors that include surface electrodes. The surface electrodes are arranged to provide one or both of monitoring surface electrocardiograms (ECGs) and delivering cardioverter and defibrillator shock therapy.

Data collected by an ambulatory medical device can be used to generate a report on the health of the patient. Some ambulatory medical devices are designed to communicate information to a second separate device that generates the report. For example, an implantable device typically communicates information wirelessly to an external device. If the device is wearable, the report can be generated by the ambulatory device itself.

Because the amount of available storage in an ambulatory medical device may be limited, information collected by the ambulatory medical device may be limited to simple data entry form. Reports generated from the collected data may be difficult for the reader to comprehend.

### OVERVIEW

This document relates generally to systems, devices, and methods that provide therapy to a patient or subject. In particular it relates to, systems, devices, and methods for automatic report generation by such devices.

A system example includes a physiologic sensing circuit configured to generate physiologic data for a subject and a processing circuit. The processing circuit includes a data parsing circuit configured to parse the physiologic data to identify one or more physiologic events and a report generation circuit. The report generation circuit is configured to associate narrative text with the identified physiologic events according to a text generating rule and generate a narrative report for the identified physiologic events using the narrative text.

This section is intended to provide an overview of subject matter of the present patent application. It is not intended to provide an exclusive or exhaustive explanation of the invention. The detailed description is included to provide further information about the present patent application.

International published patent application No. WO 97/32272 discloses method for generating sentences of text for selected medical findings in an electronic medical diagnostic system.

WO97/26043 discloses a system for storing patient data in an implantable medical device and retrieving and reconstituting the patient data from the medical device through the use of a device programmer.

U.S. published patent application No. 2005/0273362 A1 discloses a method and a system for generating medical narrative.

U.S. published patent application No. 2007/0169021 A1 discloses a report generation system including a first transformation processor for processing individual coded template text representative phrases for accommodating corresponding data items to provide corresponding formatted individual phrases.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, the various examples discussed in the present document.
FIG. 1 is an illustration of an example of portions of a system that uses an ambulatory medical device.
FIG. 2 is an illustration of portions of another system that uses an ambulatory medical device.
FIG. 3 is a flow diagram of an example of a method of automatically generating a narrative report using a medical device.
FIG. 4 shows an example of process flow to generate a narrative report.
FIG. 5 shows an example of narrative text for a narrative report.
FIG. 6 shows an example of narrative text for a narrative report in a language different from the example in FIG. 5.
FIG. 7 is a block diagram of an example of portions of an electronic system for generating a narrative report that explains physiologic events of a subject.
FIG. 8 is a block diagram of another example of portions of an electronic system for generating a narrative report.
FIGS. 9A-9B show portions of an example of a lookup table.
FIG. 10 shows a portion of an example of a set of logic rules.
FIG. 11 shows portions of an example of a decision tree.

### DETAILED DESCRIPTION

The present invention is set out in the appended claims. The embodiments, aspects or examples according to the present description that do not fall within the scope of said claims are provided for illustrative purposes only and do not form part of the present invention.

An ambulatory medical device (e.g., an IMD or WMD) can include one or more of the features, structures, methods, or combinations thereof described herein. For example, a cardiac monitor or a cardiac stimulator may be implemented to include one or more of the advantageous features or processes described below. Such a device may be implemented to provide a variety of therapeutic or diagnostic functions.

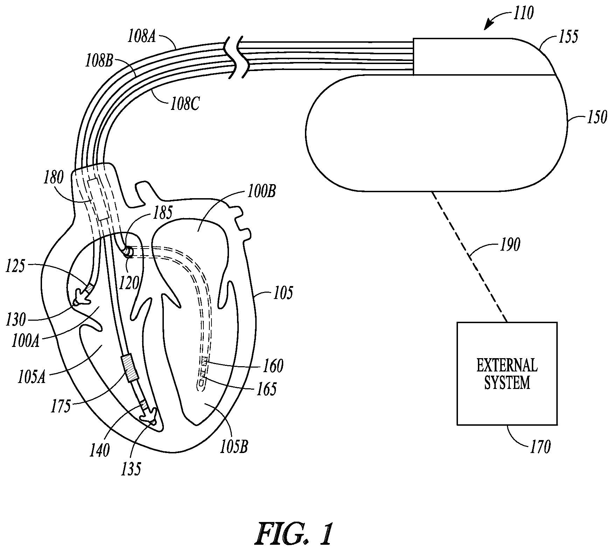

FIG. 1 is an illustration of an example of portions of a system that uses an IMD 110 or other ambulatory medical device that can be capable of moving about with the subject, such as chronically during activities of daily living. Examples of IMD 110 include, without limitation, a pacemaker, a defibrillator, a cardiac resynchronization therapy (CRT) device, or a combination of such devices. The system 100 also typically includes an IMD programmer or other external device 170 that communicates wireless signals 190 with the IMD 110, such as by using radio frequency (RF) or other telemetry signals.

The IMD 110 can be coupled by one or more leads 108A-C to heart 105. Cardiac leads 108A-C include a proximal end that is coupled to IMD 110 and a distal end, coupled by electrical contacts or "electrodes" to one or more portions of a heart 105. The electrodes typically deliver cardioversion, defibrillation, pacing, or resynchronization therapy, or combinations thereof to at least one chamber of the heart 105. The electrodes may be electrically coupled to sense amplifiers to sense electrical cardiac signals.

Sensed electrical cardiac signals can be sampled to create an electrogram. An electrogram can be analyzed by the IMD and/or can be stored in the IMD and later communicated to an external device where the sampled signals can be displayed for analysis.

Heart 105 includes a right atrium 100A, a left atrium 100B, a right ventricle 105A, a left ventricle 105B, and a coronary sinus 120 extending from right atrium 100A. Right atrial (RA) lead 108A includes electrodes (electrical contacts, such as ring electrode 125 and tip electrode 130) disposed in an atrium 100A of heart 105 for sensing signals, or delivering pacing therapy, or both, to the atrium 100A.

Right ventricular (RV) lead 108B includes one or more electrodes, such as tip electrode 135 and ring electrode 140, for sensing signals, delivering pacing therapy, or both sensing signals and delivering pacing therapy. Lead 108B optionally also includes additional electrodes, such as for delivering atrial cardioversion, atrial defibrillation, ventricular cardioversion, ventricular defibrillation, or combinations thereof to heart 105. Such electrodes typically have larger surface areas than pacing electrodes in order to handle the larger energies involved in defibrillation. Lead 108B optionally provides resynchronization therapy to the heart 105. Resynchronization therapy is typically delivered to the ventricles in order to better synchronize the timing of depolarizations between ventricles.

The IMD 110 can include a third cardiac lead 108C attached to the IMD 110 through the header 155. The third cardiac lead 108C includes electrodes 160 and 165 placed in a coronary vein lying epicardially on the left ventricle (LV) 105B via the coronary vein. The third cardiac lead 108C may include anywhere from two to eight electrodes, and may include a ring electrode 185 positioned near the coronary sinus (CS) 120.

Lead 108B can include a first defibrillation coil electrode 175 located proximal to tip and ring electrodes 135, 140 for placement in a right ventricle, and a second defibrillation coil electrode 180 located proximal to the first defibrillation coil 175, tip electrode 135, and ring electrode 140 for placement in the superior vena cava (SVC). In some examples, high-energy shock therapy is delivered from the first or RV coil 175 to the second or SVC coil 180. The combination of electrodes used in shock therapy is sometimes called a shock channel or shock vector because the combination of electrodes can result in delivery of therapy in a particular direction. In some examples, the SVC coil 180 is electrically tied to an electrode formed on the hermetically-sealed IMD housing or can 150. This improves defibrillation by delivering current from the RV coil 175 more uniformly over the ventricular myocardium. In some examples, the therapy is delivered from the RV coil 175 only to the electrode formed on the IMD can 150. In some examples, the coil electrodes 175, 180 are used in combination with other electrodes for sensing signals.

Note that although a specific arrangement of leads and electrodes are shown the illustration, an IMD can be configured with a variety of electrode arrangements, including transvenous, endocardial, and epicardial electrodes (i.e., intrathoracic electrodes), and/or subcutaneous, non-intrathoracic electrodes, including can, header, and indifferent electrodes, and subcutaneous array or lead electrodes (i.e., non-intrathoracic electrodes). The present methods and systems will work in a variety of configurations and with a variety of electrodes. Other forms of electrodes include meshes and patches which can be applied to portions of heart 105 or which can be implanted in other areas of the body to help "steer" electrical currents produced by IMD 110.

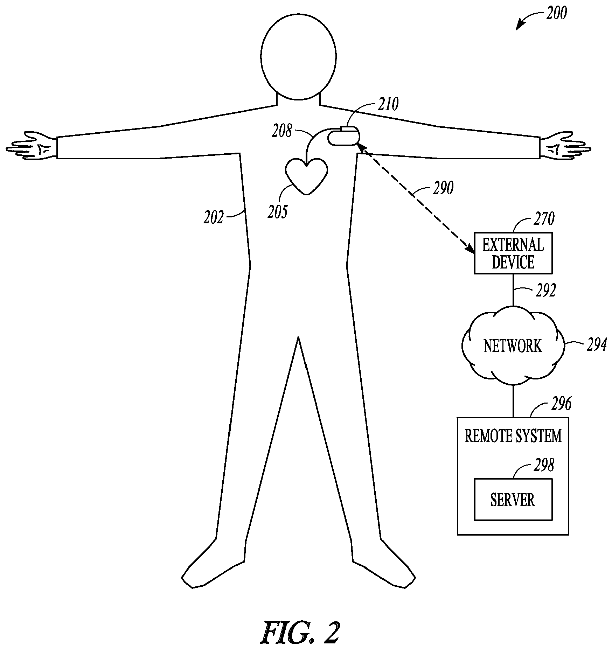

FIG. 2 is an illustration of portions of another system 200 that uses an IMD 210 to provide a therapy to a patient 202. The system 200 typically includes an external device 270 that communicates with a remote system 296 via a network 294. The network 294 can be a communication network such as a phone network or a computer network (e.g., the internet). In some examples, the external device includes a repeater and communicated via the network using a link 292 that may be wired or wireless. In some examples, the remote system 296 provides patient management functions and may include one or more servers 298 to perform the functions.

As noted previously, episode history reports can be automatically generated using information collected by ambulatory medical devices. The reports can include a history of one or more health-related episodes of the patient. These episodes can involve physiologic events experienced by the patient during a time period of interest. Physiologic data can be collected during the episode by the ambulatory device using sensors incorporated into the device or using sensors in signal communication with the device.

However, a clinician may have difficulty in comprehending these episode history reports. For instance, the clinician may have difficulty relating the episode data to the algorithms performed by the device, or the clinician may have difficulty in determining the relationships of several physiologic events that occur during one or more of the episodes. This may result in the clinician having difficulty in tailoring parameters to the patient, in mistakes being made in programming the ambulatory medical device, and in inappropriate therapy being provided to the patient.

It would be desirable for the automatic report to include a narrative description or a natural language description of episode histories based on the data collected by the ambulatory medical device. This natural language can be language a clinician would use to express physiologic events of the patient, rather than a report with device-based terms and abbreviations. A report in narrative form can improve a clinician's understanding of decisions made by the ambulatory medical device and interactions of the features of the device. This can improve the tailoring of the device to the patient and may reduce inappropriate therapy.

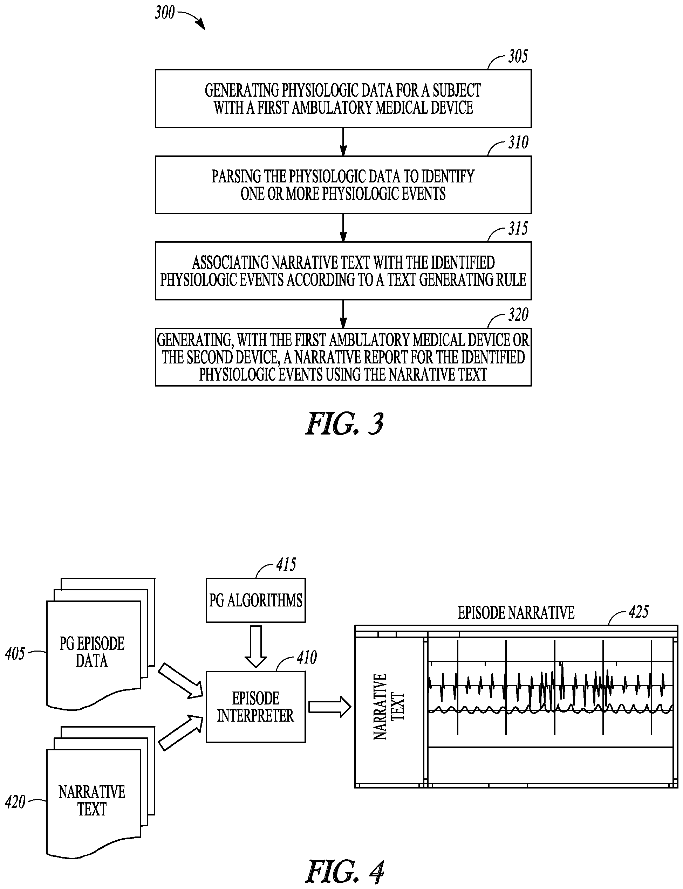

FIG. 3 is a flow diagram of an example of a method 300 of automatically generating a narrative report using a medical device. The narrative report can be a narrative language description of events related to the health of the patient or subject. At block 305, physiologic data for the subject is generated with a first ambulatory medical device. At block 310, the physiologic data is parsed to identify one or more physiological events of the subject.

The ambulatory medical device can be a wearable device such as a WCD, or a wearable monitoring device. In this case, the data can be parsed by the ambulatory medical device. If the ambulatory medical device is an implantable medical device, such as an ICD or pacemaker, the physiological data can be collected by the ambulatory medical device and communicated to a separate device, such as a device programmer or a repeater. A repeater can then pass the data on to a network such as a computer network or a cellular phone network to be received by a server or cell phone. The data can then be parsed by the programmer, server, or an application on the cell phone.

In some examples, the ambulatory medical device includes a cardiac signal sensing circuit and the physiologic data to be parsed can include electrograms and markers associated with the electrograms. If the ambulatory medical device includes an accelerometer or other motion sensor, the physiologic data can include patient activity data. If the ambulatory medical device includes a respiration sensor, the physiologic data can include respiratory data. Other examples include heart sound data and intracardiac and transthoracic impedance data.

The physiologic event can be events related to the hemodynamic function of the subject. Examples of physiologic events include, among other things, a change in heart rate, an episode of an abnormally slow heart rate or bradycardia, and an episode of an abnormally fast heart rate or tachyarrhythmia. Tachyarrhythmia includes ventricular tachycardia (VT) which originates from the ventricles. Tachyarrhythmia also includes rapid and irregular heart rate, or fibrillation, including ventricular fibrillation (VF). Abnormally rapid heart rate can also be due to supraventricular tachycardia (SVT). SVT includes arrhythmias such as atrial tachycardia, atrial flutter, and atrial fibrillation. Other examples of physiologic events include an episode of ischemia, a change in status of heart failure (HF) of the patient, and sinus tachycardia. Sinus tachycardia or ST is a normal response to exercise or an elevated emotional state for example. If the ambulatory medical device provides therapy to the patient, the physiologic event can be therapy delivered to the patient in response to the detected bradycardia, tachyarrhythmia, ischemia, and change in HF.

At block 315, narrative text is associated with the identified physiologic events according to a text generating rule. The text generating rule can include, among other things, a decision tree, a lookup table, or a specified set of logic rules. At block 320, a narrative report for the identified physiologic events is generated using the narrative text. The narrative report can be generated with the ambulatory medical device or a second separate device.

FIG. 4 shows an example of process flow to generate a narrative report. In the example, physiologic episode data 405 is collected using a pulse generator (PG) such as a pacemaker, cardioverter/defibrillator, or combination pacemaker cardioverter/defibrillator. The episode data 405 is communicated (e.g., by wireless telemetry) to a separate device having an episode interpreter 410. The episode interpreter 410 parses the episode data to identify physiologic events in the data. The episode interpreter 410 may also receive information as to the type of PG and may use information related to the type or types of algorithms 415 performable by the PG type in identifying the physiologic events. For example, the episode interpreter 410 may determine from the PG type that the PG uses a tachyarrhythmia detection enhancement to rate zone detection, such as ventricular rate being greater than the atrial rate (Vrate > Arate) for example. The episode interpreter 410 may parse the episode data for Vrate being greater than Arate before interpreting an event as tachyarrhythmia.

The episode interpreter 410 then pulls in narrative text 420 to form into a report that is a narrative of the episode 425. The narrative text 420 may include text strings that correspond to events identified during the parsing of data. Some examples of text strings are shown below in Table 1. The text strings are accumulated and formatted to generate a narrative of the episode 425.

As shown in the example, the narrative of the episode 425 can include graphical representations of the physiologic data. Narrative text for the episode or episodes is shown displayed to the left of the graphical representation. The narrative report can be displayed on the separate device, sent to a printer to generate a printout of the narrative, or saved to a file (e.g., a disk or a *.pdf) for later access and viewing or for including the narrative report in electronic mail (e-mail).

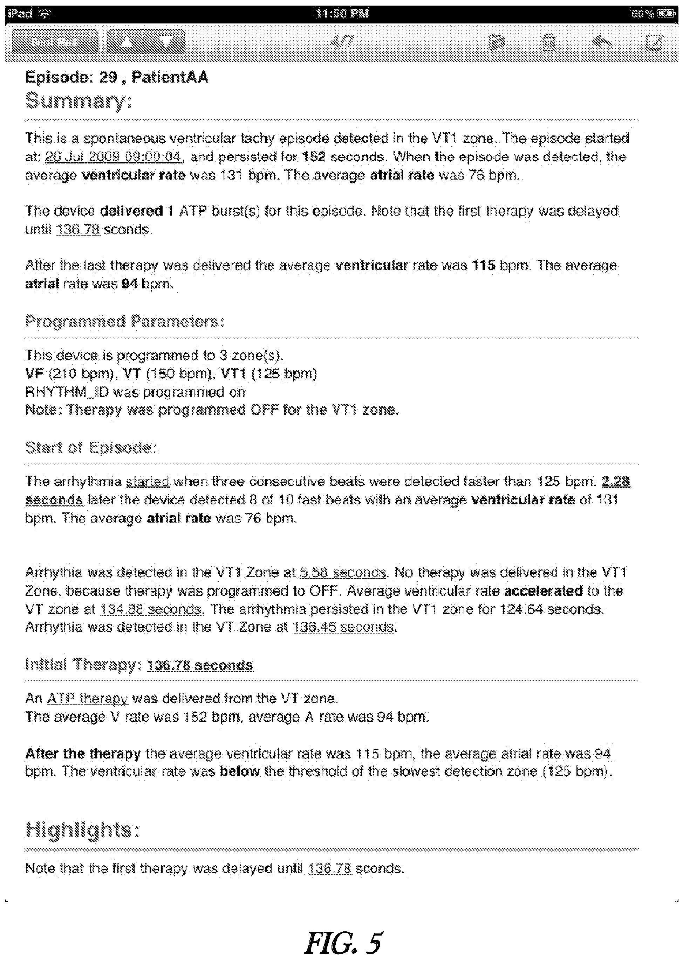

FIG. 5 shows an example of narrative text for a narrative report. Note that the first paragraph of the report explains in narrative language that the patient experienced an episode of ventricular tachycardia, instead of merely providing a marker in the graphical portion for VT. In some examples the text and graphical representations are interactive. For instance, the text can include a time or an event in the episode and clicking on the text for the time or event changes the graphical representation or an indication on the graphical representation to the specified time or event. As shown in the text example of FIG. 5, clicking on the text for "started" may change the graphical representation to show the arrhythmia start while clicking on the time text "136.78" seconds changes the graphical representation to 136.78 seconds after the episode start.

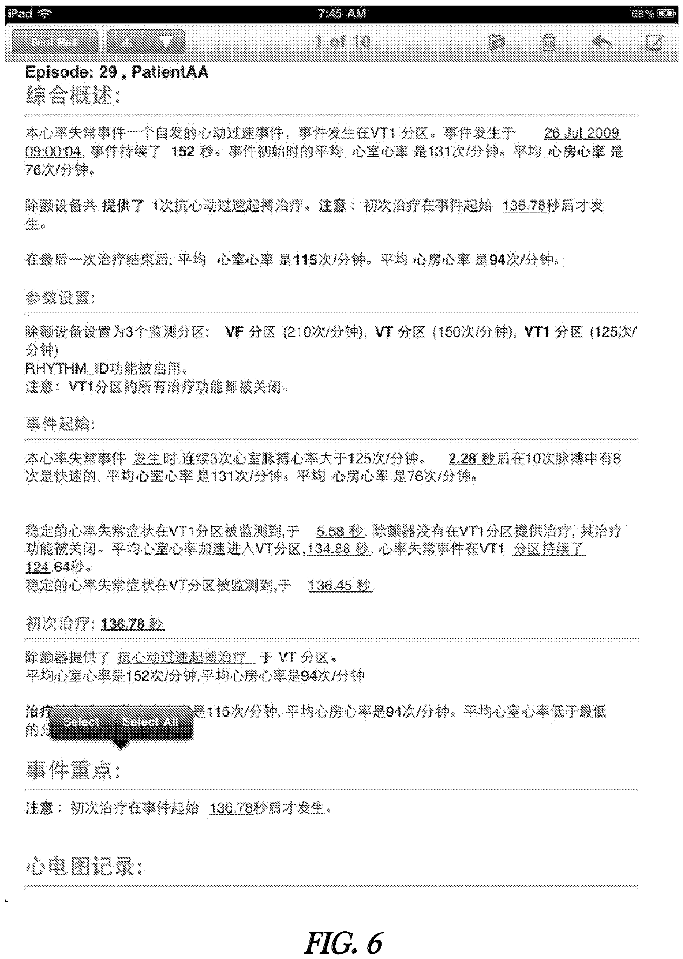

FIG. 6 shows an example of narrative text for a narrative report in a language different from the example in FIG. 5. The device generating the report may include selectable screen options for generating the narrative report in different languages.

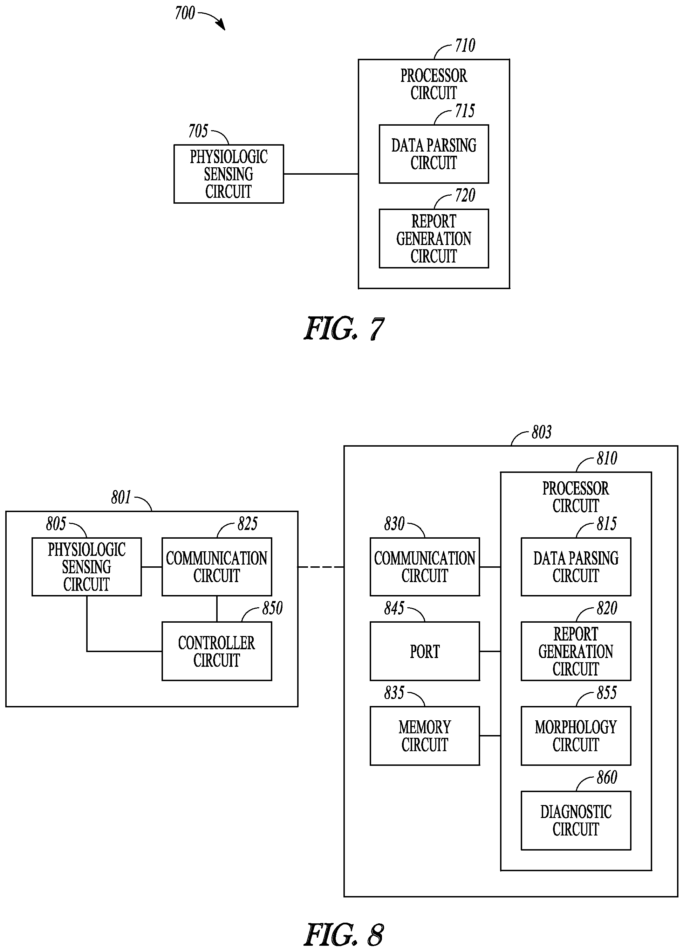

FIG. 7 is a block diagram of an example of portions of an electronic system for generating a narrative report that explains physiologic events of a subject. The system 700 includes a physiologic sensing circuit 705 and a processor circuit 710. The physiologic sensing circuit 705 generates physiologic data associated with the subject. The processor circuit 710 can include a microprocessor, a digital signal processor, application specific integrated circuit (ASIC), or other type of processor, interpreting or executing instructions in software modules or firmware modules. The processor circuit 710 includes other circuits or sub-circuits to perform the functions described. These circuits may include software, hardware, firmware or any combination thereof. Multiple functions can be performed in one or more of the circuits or sub-circuits as desired.

The processor circuit 710 includes a data parsing circuit 715 and a report generation circuit 720. The data parsing circuit 715 parses the physiologic data to identify one or more physiologic events. The report generation circuit 720 associates narrative text with the identified physiologic events according to a text generating rule, and generates a narrative report for the identified physiologic events using the narrative text.

According to some examples, the physiologic sensing circuit 705 and the processor circuit 710 are included in the same medical device, such as a wearable medical device. The physiologic sensing circuit 705 can include skin surface electrodes to obtain ECG data. The data parsing circuit 715 can parse the ECG data to identify cardiac depolarizations. Using the identified depolarizations, the data parsing circuit 715 can identify heart rate and cardiac arrhythmias such as tachyarrhythmia and bradycardia. In some examples, the physiologic sensing circuit 705 can include a respiration sensor to detect motion (such as by an accelerometer for example) of the chest cavity of the subject to detect breathing of the subject and generate respiration data. The data parsing circuit 715 may parse the ECG data and respiration data to determine an HF status of the subject.

According to some examples, the physiologic sensing circuit and the processor circuit are included in separate medical devices. FIG. 8 is a block diagram of another example of portions of an electronic system for generating a narrative report. The system 800 includes a first ambulatory medical device 801 and a second device 803. The ambulatory medical device 801 includes a physiologic sensing circuit 805 that generates physiologic data for a subject, and a communication circuit 825 to communicate the physiologic data wirelessly to a separate device.

The second device 803 includes a communication circuit 830 to receive the physiologic data and a processor circuit 810. The processor circuit 810 includes a data parsing circuit 815 and a report generation circuit 820. In certain examples, the second device 803 can be a programmer for the ambulatory medical device 801. In certain examples, the second device 803 can be a laptop computer, tablet computer, or cell phone, and the report generation circuit 820 may execute an application to generate a narrative report.

In some examples, physiologic data is received by the second device 803 from a third device (e.g., another ambulatory medical device or a server). The data parsing circuit 815 is configured to associate physiologic data from the third device with the physiologic data communicated from the ambulatory medical device, and the report generation circuit 820 is configured to associate narrative text with the identified physiologic events and the associated physiologic data.

The report generation circuit 820 includes a text generating rule to associate narrative text to the physiologic data collected by the physiologic sensing circuit 805 of the ambulatory medical device 801. In some examples, the text generating rule includes a lookup table. Entries of the lookup table can be linked to narrative text. The lookup table may be stored in a memory circuit 835 that can be integral to the processor circuit 810 or can be communicatively coupled to the processor circuit 810. The communicative coupling allows the processor to communicate with the memory even though there may be intervening circuitry.

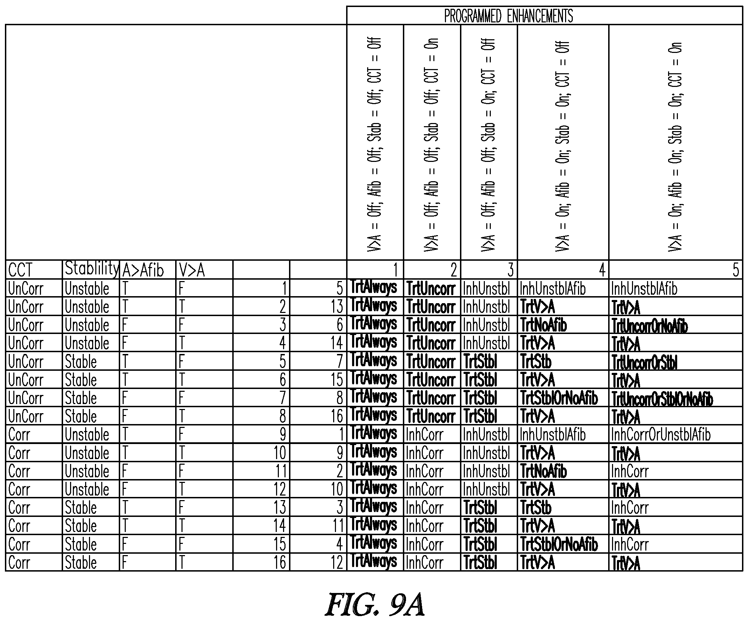

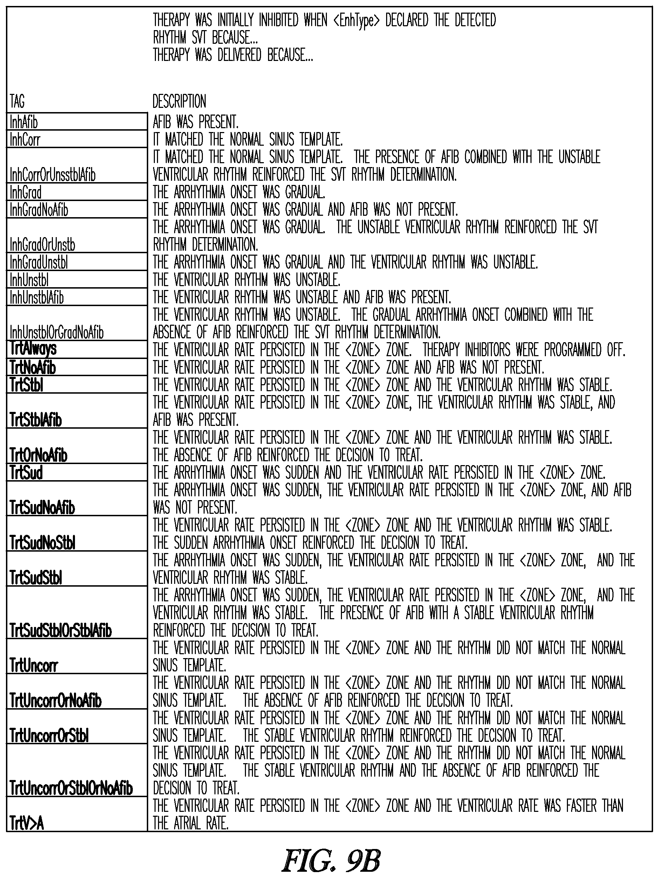

FIGS. 9A-9B show portions of an example of a lookup table used to associate narrative text to the physiologic data. The portion of the lookup table in FIG. 9A is used to determine a tag that is then used as an index or link to narrative text, such as the portion of narrative text shown in FIG. 9B. In the example, the ambulatory medical device 801 is an ICD and includes the physiologic sensing circuit 805 includes a cardiac signal sensing circuit. Rows of the table in FIG. 9A correspond to characteristics of a detected heart rhythm in the sensed physiologic data. The characteristics can be determined by the data parsing circuit 815. In some examples, the data parsing circuit 815 can be included in the ambulatory medical device 801 and the determined characteristics of the detected rhythm are communicated to the second device 803.

The columns for the detected rhythm correspond to detection enhancements to rate-based detection for an ICD. The columns indicate whether the rhythm during an episode correlated to a template rhythm, whether the rhythm was stable or unstable, whether the atrial rate (A) was greater than a threshold rate used to detect atrial fibrillation (Afib), and whether the ventricular rate (V) was greater than the atrial rate.

The table in FIG. 9A also includes columns that correspond to the detection enhancements being programmed "on" or "off." Thus, the narrative text can indicate a relationship between events detected in the heart rhythm and programmed parameters of the ambulatory medical device 801. For instance, if the rhythm is uncorrelated, is unstable, the atrial rate indicates atrial fibrillation, and the ventricular rate is not greater than the atrial rate, the first row of the table results in the **"TrtAlways"** (treat always, or initiate therapy) tag being selected if all of the detection enhancements are programmed off. If the stability enhancement is programmed "on" (the third programmed enhancements column), the first row of the table results in the "InhUnstbl" (inhibit unstable) tag being selected.

A selected tag is then indexed into the table of FIG. 9B. The tags in bold indicate a case where a decision by an ambulatory medical device (e.g., the ambulatory medical device 801 in FIG. 8) results in therapy (e.g., cardioversion or defibrillation) being delivered by the medical device, and tags in non-bold correspond to a decision that results in therapy being inhibited. The selected tags can then index text that can be linked with the narrative text at the top of the table in FIG. 9B. For the **TrtAlways** tag, the narrative text string "Therapy was delivered because the ventricular rate persisted in the <zone> zone. Therapy inhibitors were programmed Off," is generated using the lookup table. The text field <zone> would include text to indicate a tachyarrhythmia zone that included the detected rate, such as ventricular tachycardia ("VT zone") or ventricular fibrillation ("VF zone") for example. For the InhUnstbl tag, the narrative text string "Therapy was initially inhibited when Stability declared the detected rhythm SVT because the ventricular rhythm was unstable," is generated using the lookup table.

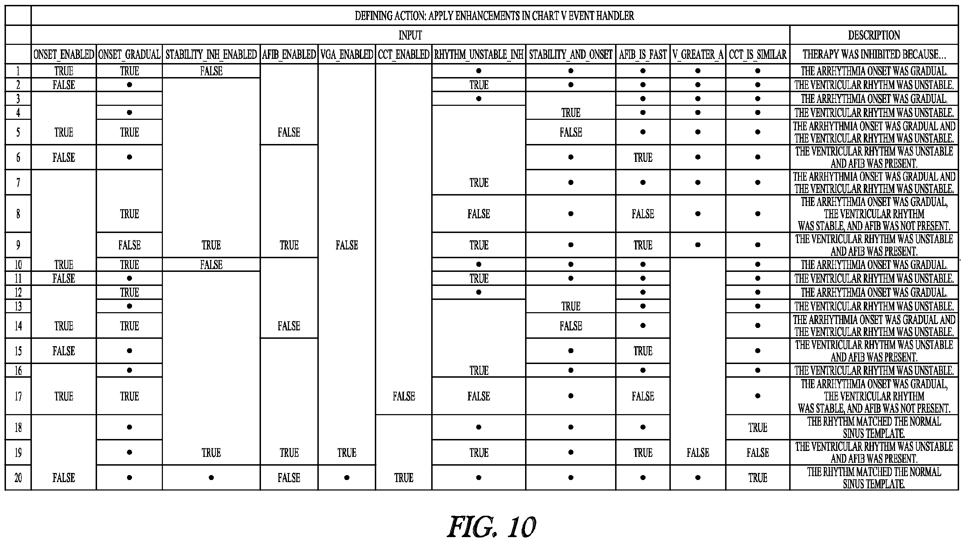

In some examples, the text generating rule includes a specified set of logic rules. Narrative text is linked to the results or outcome of the logic rules. FIG. 10 shows a portion of an example of a set of logic rules used to associate narrative text to the physiologic data. In the example shown, the logic rules are shown in the form of a table. In certain examples, the logic rules can be implanted as a series of IF-THEN statements. In the example in the Figure, the logic rules include an indication (e.g., true or false) of whether a certain condition is detected in the physiologic data. In some examples, the logic rules also include indications of whether certain parameters are programmed on or off in the ambulatory medical device. The logic rule in the first row of the Figure is valid when the onset detection enhancement is enabled, the detected onset of the rhythm was gradual, and the detection enhancements for stability, atrial fibrillation, V rate greater than A rate, and correlation were all disabled or programmed off. When the logic rule of the row is valid, the narrative text string "Therapy was inhibited because the arrhythmia onset was gradual," (as shown in the right column) is generated using the logic rules.

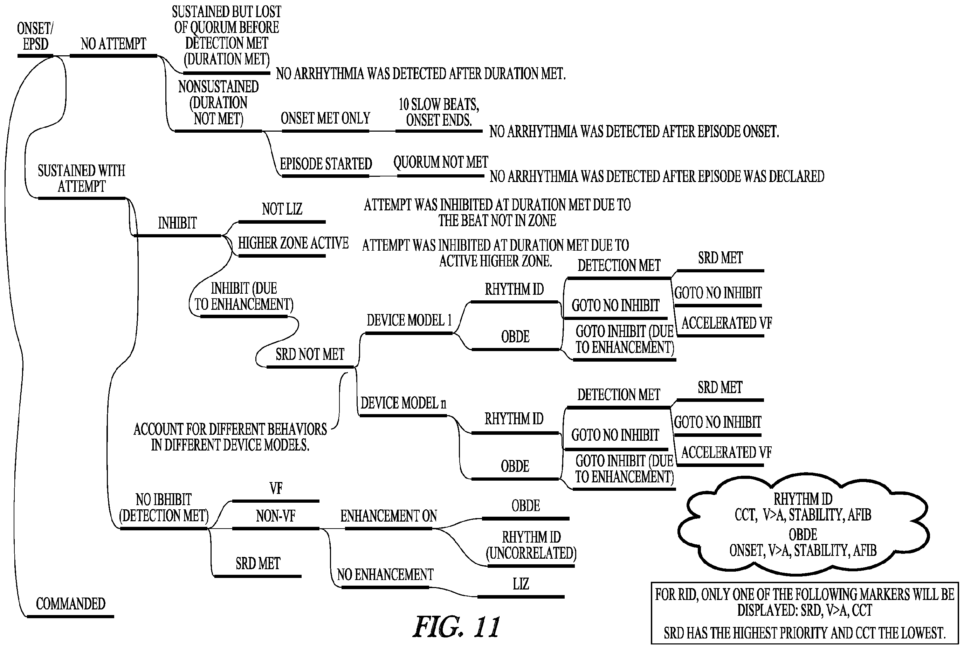

In some examples, the text generating rule can be a decision tree and the report generation circuit 820 associates narrative text with the identified physiologic events according to at least the decision tree. FIG. 11 shows portions of an example of a decision tree used to associate narrative text to the physiologic data. The example shown corresponds to a portion in which an onset of an episode of tachyarrhythmia is detected in the physiologic data. The three main branches of the decision tree shown correspond to the ambulatory medical device making no attempt to convert the rhythm of the detected episode, to the episode being sustained after an attempt to convert the episode, and an attempt to convert the episode as a result of a command received by the ambulatory medical device.

Nodes of the decision tree are linked to narrative text. For instance, for the second sub-branch form the top of the Figure, if no attempt was made by the ambulatory medical device to convert the rhythm because the duration was not sustained (SRD not met), the detection requirement for onset was the only requirement met, and onset was declared ended after ten slow beats, the narrative text "No arrhythmia was detected after the episode onset was detected" is associated to the identified physiologic event (of tachyarrhythmia onset) using the decision tree. In another example, if the onset episode was sustained after a conversion attempt, but a subsequent conversion attempt was inhibited due to the last heart beat not being in the detection for delivery of therapy (not LIZ), then the narrative text "Attempt was inhibited at duration met due to a beat not being in the detection zone" is associated to the identified physiologic event using the decision tree.

The narrative text may be included in the decision tree itself (e.g., at the end of the sub-branches) or the decision tree may include links to narrative text in a stored table, such as narrative text shown in Table 1. The portion of the decision tree in the example also shows how useful a narrative report can be to a clinician. The generated narrative report explains events in plain language rather than in terms of industry jargon such as LIZ (Last beast was In the detection Zone to deliver therapy), SRD (sustained rhythm duration) met, or OBDE (one button detection enhancement) enabled.

In some examples, at least a portion of the text generating rule is specific to a model type of the ambulatory medical device. For instance, the decision tree portion in FIG. 11 shows sub-branches specific to capabilities of Device Model 1 through Device Model *n.* In Fig. 9A, portions of the lookup table can correspond to specific device model types, and in FIG. 10, a logic rule can include an entry for a device model type (e.g., Device Model 1 = true or false).

Returning to FIG. 8, in some examples the ambulatory medical device 801 includes a controller circuit 850. The controller circuit 850 can be a processor circuit or the controller circuit 850 can be a sequencer. A sequencer refers to a state machine or other circuit that sequentially steps through a fixed series of steps to perform one or more functions. The steps are typically implemented in hardware or firmware. The controller circuit 840 initiates communication of one or more indications of a decision or adjudication, made by the ambulatory medical device 801, in association with the physiologic data.

The report generation circuit 820 associates narrative text with the decision indications and generates a narrative report for the identified one or more physiologic events and the indications of decisions made by the first medical device using the narrative text. For instance, the data parsing circuit 815 may identify an episode of ventricular tachyarrhythmia using (e.g., by parsing) the physiologic data. The report generation circuit 820 is configured to include, in the generated narrative report, an indication of a decision whether to treat the episode of ventricular tachyarrhythmia using device-based therapy. For example, the report generation circuit 820 may include the lookup table of table of FIG. 9B and indicate in the narrative text a device-based decision as to whether therapy was delivered or inhibited and the reason or reasons why. In another example, the report generation circuit 820 may include an implementation of the decision tree of FIG. 11 and indicate in the narrative text whether therapy was attempted or inhibited according to the branches of the decision tree.

In some examples, the second device 803 receives a rationale for the adjudication or decision made by the first ambulatory medical device 801 in association with the communicated physiologic data. The report generation circuit 820 associates narrative text with the rationale for the adjudication or decision, and generates, using the narrative text, a narrative report according to the identified physiologic events, the indication of the adjudication, and the rationale for the adjudication or decision.

In some examples, the report generation circuit 820 relates a decision made by the first ambulatory medical device to a specified device parameter setting that influenced the decision. For instance, the communicated physiologic data may include the results of one or more tachyarrhythmia detection enhancements of the first ambulatory medical device. In certain examples, the data can include indications of whether the specific detection enhancements were enabled during a detected tachyarrhythmia episode. The report generation circuit 820 is configured to associate narrative text with the results and include the narrative text in the generated narrative report. Examples of detection enhancements for correlation, rhythm stability, atrial rate, and ventricular rate greater than atrial rate are shown in the examples, FIGS. 9A, 9B, 10, and 11.

In some examples, the data parsing circuit 815 may identify an episode of bradycardia using the physiologic data. The report generation circuit 820 includes, in the generated narrative report, an indication of a decision whether to treat the episode of bradycardia using device-based therapy.

As explained previously, the physiologic sensing circuit 805 can include a cardiac signal sensing circuit to generate the physiologic data. In some examples, the physiologic sensing circuit 805 includes a heart sound sensing circuit. Heart sounds are associated with mechanical vibrations from activity of a patient's heart and the flow of blood through the heart. Heart sounds recur with each cardiac cycle and are separated and classified according to the activity associated with the vibration. The first heart sound (S1) is the vibrational sound made by the heart during tensing of the mitral valve. The second heart sound (S2) marks the beginning of diastole. The third heart sound (S3) and fourth heart sound (S4) are related to filling pressures of the left ventricle during diastole.

A heart sound sensing circuit produces an electrical signal which is representative of mechanical activity of a patient's heart. Regional shortening causes changes in the heart sounds detectable with a heart sound sensor. A description of systems and methods for sensing wall motion is found in the commonly assigned, co-pending U.S. Patent Application, Serial No. 11/135,985, entitled "Systems and Methods for Multi-Axis Cardiac Vibration Measurements," filed May 24, 2005. The physiologic data can include heart sound data. The data parsing circuit 815 may parse the heart sound data to detect an episode of myocardial ischemia. The report generation circuit 820 may associate narrative text with an indication of ischemia and may indicate a device-based decision based on the detection of ischemia, such as initiating a regimen of protection pacing for example.

In some examples, the physiologic sensing circuit 805 includes a cardiac impedance sensing circuit. Cardiac impedance changes measure changes in chamber volumes. Regional changes in cardiac relaxation may be measured using measurements of cardiac impedance using an impedance sensing circuit. Similarly, the strength of contraction may be inferred from changes in the rate of decrease of cardiac impedance during cardiac contraction. Systems and methods to measure intracardiac impedance are described in Citak et al., U.S. Pat. No. 4,773,401, entitled "Physiologic Control of Pacemaker Rate Using Pre-Ejection Interval as the Controlling Parameter," filed August 21. The data parsing circuit 815 may parse cardiac impedance data to detect an episode of ischemia and the report generation circuit 820 may associate narrative text with one or more an indication of the ischemia a device-based decision based on the detection of ischemia.

In some examples, the physiologic sensing circuit 805 includes a physical activity sensing circuit, such as an accelerometer for example, to provide a signal representative of activity of the subject. The report generation circuit 820 may associate narrative text with an indication of patient activity and may indicate a device-based decision based on determined activity of the patient, such as an increase in paced heart rate for example.

In some examples, the physiologic sensing circuit 805 includes a respiration sensing circuit to provide a signal representative of respiration of the subject. An example of an implantable respiration sensor is a transthoracic impedance sensor to measure minute respiration volume. An approach to measuring transthoracic impedance is described in Hartley et al., U.S. Patent No. 6,076,015, "Rate Adaptive Cardiac Rhythm Management Device Using Transthoracic Impedance," filed February 27, 1998. The physiologic data can include respiration data such as breathing tidal volume for example. The data parsing circuit 815 may use one or more of respiration data, physical activity data, and cardiac depolarization data to determine a change in HF status of the subject. The report generation circuit 820 may associate narrative text with an indication of the change in HF status of the patient as well as any device-based decision based on the HF status, such as a change in resynchronization pacing therapy for example.

In some examples, the data parsing circuit 815 provides an adjudication of a physiologic event. For instance, the data parsing circuit 815 may identify cardiac arrhythmia (e.g., an episode of bradycardia or tachyarrhythmia) from the physiologic data and the report generation circuit 820 associates narrative text with the adjudicated event.

In some examples, the second device 803 includes a port 845. In some examples, the port 845 includes a communication port or "comm" port to receive information from a third device. In certain examples the port is a wired interface (e.g., a Universal Serial Bus or USB), and in certain examples the port 845 is a wireless interface. In some examples, the port 845 includes a user interface to receive information entered by a user. The second device 803 may receive an indication of adjudication of an identified physiologic event via the port 845. The report generation circuit 820 is configured to change the content of the generated narrative report, or the amount of detail in the generated narrative report, related to the identified physiologic event based on the received adjudication.

For instance, the data parsing circuit 815 may identify a physiologic event in the physiologic data as SVT. The second device 803 may receive an indication of adjudication related to the episode of SVT and the report generation circuit 820 changes an amount of detail in the generated narrative report related to SVT detection enhancements. The adjudication may be received from a separate device or via a user interface. In another example, the data parsing circuit 815 may identify a physiologic event in the physiologic data as VT and an indication of adjudication of the episode is received via the port 845 that the episode is SVT. The report generation circuit 820 changes the content of the narrative report accordingly.

In some examples, the physiologic sensing circuit 805 produces samples of a sensed physiologic signal as at least a portion of the physiologic data. The physiologic sensing circuit 805 may include a sampling circuit to generate digital valued samples of one or more of a sensed cardiac signal, a heart sound signal, a respiration signal, an impedance signal, or an activity signal. The port 845 may receive an indication (e.g., via a user interface) of a specified segment of the sensed and sampled physiologic signal for which to generate the narrative report.

In some examples, the second device 803 includes a display (not shown) to display a graphical representation of physiologic data such as is shown in FIG. 4. For instance, the physiologic sensing circuit 805 may include a cardiac signal sensing circuit. The ambulatory medical device 801 may include a controller circuit 850 that recurrently initiates sensing of electrograms using the cardiac signal sensing circuit. The report generation circuit 820 can incorporate a graphical representation of a comparison of two or more sensed electrograms in the generated narrative report. In certain examples, the report generation circuit 820 incorporates a graphical representation of one or more of one or more of a sensed cardiac signal, a heart sound signal, a respiration signal, an impedance signal, or an activity signal in the generated narrative report. The generated narrative report can be displayed, communicated to a printer to generate a printout of the narrative, or saved to a file for later access and viewing or for including the narrative report in e-mail.

According to some examples, the data parsing circuit 815 is able to determine a relationship between multiple identified physiologic events according to determined attributes of the physiologic events. In some examples, the data parsing circuit 815 is able to identify physiologic events of the same type. For example, the processor circuit 810 can include a morphology circuit 855 that identifies physiologic events for a subject using the communicated physiologic data, and compares morphology of the physiologic events. In certain examples, the morphology circuit 855 compares sensed cardiac signals to identify physiologic events of the same type. Descriptions of systems to identify physiologic events using signal morphology can be found in Hsu et al., U.S. Patent No. 6,438,410, "System and Method for Classifying Cardiac Complexes," filed May 3, 2001.

The data parsing circuit 815 can identify the physiologic events having the same type and the report generation circuit 820 can include narrative text related to a result of the morphology comparison in the generated narrative report. In some examples, the data parsing circuit 815 is able to identify physiologic events of the same type as belonging to the same event. For instance, the data parsing circuit 815 may use proximity of the identified physiologic events to determine that they are actually included in the single event. For example, multiple episodes of tachyarrhythmia may actually belong to the same single episode of tachyarrhythmia.

In some examples, the data parsing circuit identifies physiologic events of different types by parsing the physiologic data. The processor circuit 810 may include a diagnostic circuit 860 configured to determine a link, if any, between the different physiologic events. In certain examples, the diagnostic circuit 860 uses attributes such as proximity of the identified physiologic events to determine a link between the events. In certain examples, the diagnostic circuit 860 uses duration of identified physiologic events to determine that the physiologic events are related. The report generation circuit 820 includes narrative text in the generated narrative report to indicate any determined link between the different physiologic events.

Automatically generating a narrative description or a natural language description of episode histories using a medical device may reduce any difficulty a clinician may have in comprehending episode history reports, such as by indicating the relationships of several physiologic events that occur during one or more of the episodes for example. This may result in reduced difficulty for the clinician in tailoring parameters to the patient, which can reduce reliance by clinicians on technical services provided by the device manufacturer. This may also reduce mistakes being made in programming the ambulatory medical device and reduce inappropriate therapy being provided to the patient.

**Table 1**

| ***Event Start*** |
|---|
| 1. *This episode started spontaneously within 30 seconds of a commanded induction.* |
| 2. *The episode started at <start> when three consecutive beats were detected faster than <intvl> bpm.* |
| 3. *The episode started at <start> when <therapy> was commanded.* |
| |

| ***Episode Start*** |
|---|
| 1. *The arrhythmia onset was declared <Sudden*/*Gradual>, because the calculated Onset of <meas> was greater than*/*less than*/*equal to the programmed Onset threshold of <thres> <units>.* |
| |

| ***Rates*** |
|---|
| 1. *When the episode started, the ventricular rate was <vrate> bpm and the atrial rate*was *<arate> bpm.* |
| 2. *When <therapy> was commanded, the ventricular rate was <vrate> bpm and theatrial rate* was *<arate> bpm.* |
| 3. *When the device decided to attempt therapy, the ventricular rate <was*/*increased to*/*decreased to> <vrate> bpm and the atrial rate <was*/*increased to*/*decreased to> <arate> bpm.* |
| 4. *<time> seconds after therapy delivery, the ventricular rate <was*/*increased to*/*decreased to> <vrate> bpm and the atrial rate <was*/*increased to*/*decreased to> <arate> bpm.* |
| |

| ***Non-Sustained*** |
|---|
| 1. *The arrhythmia was non-sustained.* |
| |

| **No Attempt** |
|---|
| 1. *The arrhythmia was sustained but the episode spontaneously ended before therapy was indicated.* |

| **Therapy Initially Inhibited** |
|---|
| 1. *Therapy was initially inhibited when Onset*/*Stability declared the detected rhythm SVT because the arrhythmia onset was gradual.* |
| *a. The unstable ventricular rhythm reinforced the SVT rhythm determination.* |
| 2. *Therapy was initially inhibited when Onset*/*Stability declared the detected rhythm SVT because the ventricular rhythm* was *unstable.* |
| *a. The gradual arrhythmia onset combined with the absence of Afib reinforced the SVT rhythm determination.* |
| *3. Therapy was initially inhibited when Onset*/*Stability declared the detected rhythm SVT because the arrhythmia onset was gradual and the ventricular rhythm was unstable.* |
| *4. Therapy was initially inhibited when Onset*/*Stability declared the detected rhythm SVT because the ventricular rhythm was unstable and Afib was present.* |
| *5. Therapy was initially inhibited when Onset*/*Stability declared the detected rhythm SVT because the arrhythmia onset was gradual and Afib was not present.* |
| *6. Therapy was initially inhibited when Onset*/*Stability declared the detected rhythm SVT because Afib was present.* |
| |
| *7. Therapy was initially inhibited when Rhythm ID declared the detected rhythm SVT because it matched the normal sinus template.* |
| |
| *a. The presence of Afib combined with the unstable ventricular rhythm reinforced the SVT rhythm determination.* |
| *8. Therapy was initially inhibited when Rhythm ID declared the detected rhythm SVT because the ventricular rhythm was unstable and Afib was present.* |
| *9. When therapy was initially inhibited,* |
| *- the rhythm was stable*/*unstable because the measured Stability Variance of <stb> ms was <greater than*/*less thanlequal to> the Stability Threshold of <thresh> ms.* |
| *- the rhythm was determined (not) to be Afib, because at least*/*fewer than 6 of 10 beats were faster than the Afib Threshold of <thresh> bpm.* |
| *- the ventricular rate was not 10 bpm faster than the atrial rate.* |
| *- beats were (not) correlated with the normal sinus template.* |
| *- The measured RhythmMatch was <RhythmMatch>%* |
| |

| ***Therapy Delivered*** |
|---|
| *1. VF therapy was subsequently delivered after the rhythm accelerated to the VF zone.* |
| *2. Therapy was attempted because the ventricular rate persisted in the <zone> zone.* |
| *a. Therapy inhibitors were programmed Off.* |
| *3. Therapy was attempted because the ventricular rate persisted in the <zone> zone and was at least 10 bpm faster than the atrial rate.* |
| *4. Therapy was attempted because the ventricular rate persisted in the <zone> zone and the programmed Sustained Rate Duration expired after <mm:ss>, overriding therapy inhibition by <EnhType>.* |
| *5. Therapy was attempted because the arrhythmia onset was sudden and the ventricular rate persisted in the <zone> zone.* |
| *6. Therapy was attempted because the ventricular rate persisted in the <zone> zone and the ventricular rhythm was stable.* |
| *a. The absence of Afib reinforced the decision to treat.* |
| *b. The sudden arrhythmia onset reinforced the decision to treat.* |
| *7. Therapy was attempted because the arrhythmia onset was sudden, the ventricular rate persisted in the <zone> zone, and the ventricular rhythm was stable.* |
| *a. The presence of Afib with a stable ventricular rhythm reinforced the decision to treat.* |
| *8. Therapy was attempted because the ventricular rate persisted in the <zone> zone and Afib was not present.* |
| *9. Therapy was attempted because the ventricular rate persisted in the <zone> zone, the ventricular rhythm was stable, and Afib was present.* |
| *10. Therapy was attempted because the arrhythmia onset was sudden, the ventricular rate persisted in the <zone> zone, and Afib was not present.* |
| *1. Therapy was attempted because the ventricular rate persisted in the <zone> zone and the rhythm did not match the normal sinus template.* |
| *a. The absence of Afib reinforced the decision to treat.* |
| *b. The stable ventricular rhythm reinforced the decision to treat.* |
| *c. The stable ventricular rhythm and the absence of Afib reinforced the decision to treat.* |
| *2. When the device decided to attempt therapy,* |
| - *the rhythm was <stable*/*unstable> because the measured Stability Variance of <stb> ms was <greater than*/*less than*/*equal to> the Stability Threshold of <thresh> ms.* |
| - *the rhythm was determined (not) to be Afib, because at least*/*fewer than 6 of 10 beats were faster than the Afib Threshold of <thresh> bpm.* |
| - *the ventricular rate was (not) 10 bpm faster than the atrial rate.* |
| - *beats were (not) correlated with the normal sinus template.* |
| - *The measured RhythmMatch was <RhythmMatch>%* |

| ***Episode End*** |
|---|
| *1. The episode ended at <end>.* |
| *2. The episode ended at <end>, <time> seconds after therapy was delivered.* |
| *3. The episode ended at <end> due to a command from the PRM.* |
| *4. The episode ended at <end> due to programming of the V-tachy parameters.* |

These non-limiting examples can be combined in any permutation or combination.

The above detailed description includes references to the accompanying drawings, which form a part of the detailed description. The drawings show, by way of illustration, specific embodiments in which the invention can be practiced. These embodiments are also referred to herein as "examples."

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times. These computer-readable media can include, but are not limited to, hard disks, removable magnetic disks, removable optical disks (e.g., compact disks and digital video disks), magnetic cassettes, memory cards or sticks, random access memories (RAM's), read only memories (ROM's), and the like. In some examples, a carrier medium can carry code implementing the methods. The term "carrier medium" can be used to represent carrier waves on which code is transmitted.

The invention is set out in the following claims:

## Claims

1. A system comprising:
a physiologic sensing circuit (705; 805) configured to generate physiologic data for a subject, wherein the physiologic sensing circuit is included in a first implantable or
wearable medical device configured to deliver device-based therapy to a patient (210; 801);
a controller circuit (850) configured to generate an indication of a device-based therapy decision of the first implantable or wearable medical device (210; 801) as to whether therapy was delivered or inhibited; and a processor circuit (710; 810) including:
a data parsing circuit (715; 815) configured to parse the physiologic data to identify one or more physiologic events;
a report generation circuit (720; 820) configured to associate narrative text with the identified physiologic events and the device-based therapy decision according to a text generating rule,
wherein at least a portion of the text generating rule is specific to a model type of the first implantable or wearable medical device (210; 801); and
generate a narrative report for the identified physiologic events and the device-based therapy decision using the narrative text.

2. The system of claim 1, wherein the report generation circuit (720; 820) is configured to associate narrative text with the identified physiologic events according to at least one of:
a decision tree, wherein nodes of the decision tree are linked to narrative text;
a lookup table, wherein entries of the lookup table are linked to narrative text; and
a specified set of logic rules.

3. The system of any one of claims 1-2,
wherein the first implantable or wearable medical device (210; 801) includes: a communication circuit (825) configured to communicate the physiologic data to a separate device (803); and
the controller circuit (850), wherein the controller circuit (850) is configured to communicate, in association with the physiologic data, one or more indications of a decision made by the first implantable or wearable medical device (210; 801); and wherein the processor circuit (810) is included in a second device (803) that includes a communication circuit configured to receive the physiologic data, and
wherein the report generation circuit (820) is configured to: associate narrative text with the decision indications; and generate a narrative report for the identified one or more physiologic events and the indications of decisions made by the first implantable or wearable medical device (210; 801) using the narrative text.

4. The system of claim 3,
wherein the controller circuit (850) is configured to communicate a rationale for the decision made by the first implantable or wearable medical device (210; 801) in association with the physiologic data; and
wherein the report generation circuit (820) is configured to: associate narrative text with the rationale for the decision, and generate, using the narrative text, a narrative report for the identified one or more physiologic events, the indication of the decision made by the first medical device, and the rationale for the decision.

5. The system of any one of claims 3-4, wherein the report generation circuit (820) is configured to relate a decision made by the first implantable or wearable medical device (210; 801) to a specified device parameter setting that influenced the decision.

6. The system of any one of claims 3-5,
wherein the data parsing circuit (815) is configured to identify an episode of at least one of tachyarrhythmia or bradycardia using the physiologic data, and
wherein the report generation circuit (820) is configured to include, in the generated narrative report, an indication of a decision whether to treat the episode of tachyarrhythmia or bradycardia using device-based therapy.

7. The system of any one of claims 3-6, wherein the second device (803) includes a port (845) configured to receive an indication of adjudication of
an identified physiologic event, and
wherein the report generation circuit (820) is configured to change content of the generated narrative report related to the identified physiologic event based on the received adjudication.

8. The system of any one of claims 1-7, wherein the physiologic sensing circuit (705: 805) includes at least one of:
a cardiac signal sensing circuit; a heart sound sensing circuit;
respiration sensing circuit;
a cardiac impedance sensing circuit; and a physical activity sensing circuit.

9. The system of any one of claims 1 -8,
wherein the physiologic sensing circuit (705: 805) is configured to produce samples of a sensed physiologic signal as at least a portion of the physiologic data, wherein the system (700; 801, 803) includes a user interface configured to receive an indication of a specified segment of the sensed physiologic signal for which to generate the narrative report.

## Patentansprüche

1. System umfassend:
eine physiologische Erfassungsschaltung (705; 805), die konfiguriert ist, um physiologische Daten für einen Probanden zu erzeugen, wobei die physiologische Erfassungsschaltung in einer ersten implantierbaren oder tragbaren medizinischen Vorrichtung enthalten ist, die konfiguriert ist, um eine vorrichtungsbasierte Therapie an einen Patienten abzugeben (210; 801);
eine Steuerungsschaltung (850), die konfiguriert ist, um eine Angabe einer vorrichtungsbasierten Therapieentscheidung der ersten implantierbaren oder tragbaren medizinischen Vorrichtung (210; 801) dahingehend, ob die Therapie abgegeben oder unterbunden wurde, zu erzeugen; und eine Prozessorschaltung (710; 810), die folgendes beinhaltet:
eine Daten-Parsing-Schaltung (715; 815), die konfiguriert ist, um die physiologischen Daten zu parsen, um ein oder mehr physiologische Ereignisse zu identifizieren;
eine Berichterzeugungsschaltung (720; 820), die konfiguriert ist, um Erzähltext mit den identifizierten physiologischen Ereignissen und der vorrichtungsbasierten Therapieentscheidung nach einer Texterzeugungsregel zu verknüpfen;
wobei mindestens ein Teil der Texterzeugungsregel spezifisch für eine Modellart der ersten implantierbaren oder tragbaren medizinischen Vorrichtung (210; 801) ist; und
Erzeugen eines Erzählberichts für die identifizierten physiologischen Ereignisse und die vorrichtungsbasierte Therapieentscheidung unter Verwendung des Erzähltextes.

2. System nach Anspruch 1, wobei die Berichterzeugungsschaltung (720; 820) konfiguriert ist, um Erzähltext mit den identifizierten physiologischen Ereignissen nach mindestens einem der folgenden zu verknüpfen:
einem Entscheidungsbaum, in dem Knoten des Entscheidungsbaums mit Erzähltext verknüpft sind;
einer Lookup-Tabelle, in der Einträge der Lookup-Tabelle mit Erzähltext verknüpft sind; und
einer bestimmten Gruppe von Logik-Regeln.

3. System nach einem der Ansprüche 1 - 2,
wobei die erste implantierbare oder tragbare medizinische Vorrichtung (210; 801) folgendes beinhaltet: eine Kommunikationsschaltung (825), die konfiguriert ist, um die physiologischen Daten an eine separate Vorrichtung (803) zu kommunizieren; und
die Steuerungsschaltung (850), wobei die Steuerungsschaltung (850) konfiguriert ist, um zusammen mit den physiologischen Daten eine oder mehr Angaben einer Entscheidung, die von der ersten implantierbaren oder tragbaren medizinischen Vorrichtung (210; 801) getroffen wird, zu kommunizieren; und
wobei die Prozessorschaltung (810) in einer zweiten Vorrichtung (803) enthalten ist, die eine Kommunikationsschaltung beinhaltet, die konfiguriert ist, um die physiologischen Daten zu erhalten, und
wobei die Berichterzeugungsschaltung (820) konfiguriert ist, um: Erzähltext mit den Entscheidungsangaben zu verknüpfen; und einen Erzählbericht für die identifizierten ein oder mehr physiologischen Ereignisse und die Angaben von Entscheidungen, die von der ersten implantierbaren oder tragbaren medizinischen Vorrichtung (210; 801) getroffen werden, unter Verwendung des Erzähltextes zu erzeugen.

4. System nach Anspruch 3,
wobei die Steuerungsschaltung (850) konfiguriert ist, um eine Begründung für die von der ersten implantierbaren oder tragbaren medizinischen Vorrichtung (210; 801) getroffenen Entscheidung zusammen mit den physiologischen Daten zu kommunizieren; und
wobei die Berichterzeugungsschaltung (820) konfiguriert ist, um: Erzähltext mit der Begründung für die Entscheidung zu verknüpfen und unter Verwendung des Erzähltextes einen Erzählbericht für die identifizierten ein oder mehr physiologischen Ereignisse, die Angabe der Entscheidung, die von der ersten medizinischen Vorrichtung getroffen wird, und die Begründung für die Entscheidung zu erzeugen.

5. System nach einem der Ansprüche 3 - 4, wobei die Berichterzeugungsschaltung (820) konfiguriert ist, um eine Entscheidung, die von der ersten implantierbaren oder tragbaren medizinischen Vorrichtung (210; 801) getroffen wird, mit einer bestimmten Vorrichtungsparametereinstellung zu verbinden, die die Entscheidung beeinflusst hat.

6. System nach einem der Ansprüche 3 - 5,
wobei die Daten-Parsing-Schaltung (815) konfiguriert ist, um einen Schub von mindestens einer von Tachyarrhythmie oder Bradykardie unter Verwendung der physiologischen Daten zu identifizieren, und
wobei die Berichterzeugungsschaltung (820) konfiguriert ist, um in dem erzeugten Erzählbericht eine Angabe einer Entscheidung, ob der Schub der Tachyarrhythmie oder Bradykardie unter Verwendung einer vorrichtungsbasierten Therapie zu behandeln ist, aufzunehmen.

7. System nach einem der Ansprüche 3 - 6, wobei die zweite Vorrichtung (803) einen Anschluss (845) beinhaltet, der konfiguriert ist, um eine Angabe über ein Urteil eines identifizierten physiologischen Ereignisses zu erhalten, und
wobei die Berichterzeugungsschaltung (820) konfiguriert ist, um basierend auf dem erhaltenen Urteil Inhalt des erzeugten Erzählberichts zu ändern, der sich auf das identifizierte physiologische Ereignis bezieht.

8. System nach einem der Ansprüche 1 - 7, wobei die physiologische Erfassungsschaltung (705, 805) mindestens eine der folgenden Schaltungen beinhaltet:
eine Herzsignalerfassungsschaltung; eine Herzgeräuscherfassungsschaltung;
Atmungserfassungsschaltung;
eine Herzimpedanzerfassungsschaltung; und eine Schaltung zur Erfassung der körperlichen Aktivität.

9. System nach einem der Ansprüche 1 - 8,
wobei die physiologische Erfassungsschaltung (705; 805) konfiguriert ist, um Abtastwerte eines erfassten physiologischen Signals als mindestens einen Teil der physiologischen Daten zu produzieren,
wobei das System (700; 801, 803) eine Nutzerschnittstelle beinhaltet, die konfiguriert ist, um eine Angabe eines bestimmten Segments des erfassten physiologischen Signals zu erhalten, für welches der Erzählbericht zu erzeugen ist.

## Revendications

1. Système comprenant :
un circuit de détection physiologique (705 ; 805) configuré pour générer des données physiologiques pour un sujet, le circuit de détection physiologique étant contenu dans un premier dispositif médical implantable ou portable configuré pour délivrer une thérapie par dispositif à un patient (210 ; 801) ;
un circuit de contrôle (850) configuré pour générer une indication d'une décision de thérapie par dispositif du premier dispositif médical implantable ou portable (210 ; 801) indiquant si la thérapie a été délivrée ou inhibée ; et un circuit de traitement (710 ; 810) comportant :
un circuit d'analyse de données (715 ; 815) configuré pour analyser les données physiologiques afin d'identifier un ou plusieurs événements physiologiques ;
un circuit de génération de rapport (720 ; 820) configuré pour associer un texte narratif aux événements physiologiques identifiés et à la décision de thérapie par dispositif en fonction d'une règle de génération de texte,
au moins une partie de la règle de génération de texte étant spécifique d'un type de modèle du premier dispositif médical implantable ou portable (210 ; 801) ; et
générer un rapport narratif pour les événements physiologiques identifiés et la décision de thérapie par dispositif en utilisant le texte narratif.

2. Système de la revendication 1, dans lequel le circuit de génération de rapport (720 ; 820) est configuré pour associer un texte narratif aux événements physiologiques identifiés en fonction d'au moins un des éléments suivants :
une arborescence de décision, les noeuds de l'arborescence de décision étant liés à un texte narratif ;
une table de conversion, les entrées de la table de conversion étant liées à un texte narratif ; et
un ensemble spécifié de règles logiques.

3. Système de l'une quelconque des revendications 1 à 2,
dans lequel le premier dispositif médical implantable ou portable (210 ; 801) comporte : un circuit de communication (825) configuré pour communiquer les données physiologiques à un dispositif séparé (803) ; et
le circuit de contrôle (850), le circuit de contrôle (850) étant configuré pour communiquer, en association avec les données physiologiques, une ou plusieurs indications d'une décision prise par le premier dispositif médical implantable ou portable (210 ; 801) ; et
dans lequel le circuit de traitement (810) est contenu dans un deuxième dispositif (803) qui comporte un circuit de communication configuré pour recevoir les données physiologiques, et
dans lequel le circuit de génération de rapport (820) est configuré pour : associer un texte narratif aux indications de décisions ; et générer un rapport narratif pour le ou les événements physiologiques identifiés et les indications des décisions prises par le premier dispositif médical implantable ou portable (210 ; 801) en utilisant le texte narratif.

4. Système de la revendication 3,
dans lequel le circuit de contrôle (850) est configuré pour communiquer une justification logique pour la décision prise par le premier dispositif médical implantable ou portable (210 ; 801) en association avec les données physiologiques ; et
dans lequel le circuit de génération de rapport (820) est configuré pour : associer un texte narratif à la justification logique de la décision, et générer, en utilisant le texte narratif, un rapport narratif pour le ou les événements physiologiques identifiés, l'indication de la décision prise par le premier dispositif médical, et la justification logique de la décision.

5. Système de l'une quelconque des revendications 3 à 4, dans lequel le circuit de génération de rapport (820) est configuré pour associer une décision prise par le premier dispositif médical implantable ou portable (210 ; 801) à un réglage de paramètre de dispositif spécifié qui a influencé la décision.

6. Système de l'une quelconque des revendications 3 à 5,
dans lequel le circuit d'analyse de données (815) est configuré pour identifier un épisode de tachyarythmie et/ou de bradycardie en utilisant les données physiologiques, et
dans lequel le circuit de génération de rapport (820) est configuré pour inclure, dans le rapport narratif généré, une indication d'une décision de traiter ou non l'épisode de tachyarythmie ou de bradycardie en utilisant une thérapie par dispositif.

7. Système de l'une quelconque des revendications 3 à 6, dans lequel le deuxième dispositif (803) comporte un port (845) configuré pour recevoir une indication de jugement d'un événement physiologique identifié, et
dans lequel le circuit de génération de rapport (820) est configuré pour modifier le contenu du rapport narratif généré associé à l'événement physiologique identifié sur la base du jugement reçu.

8. Système de l'une quelconque des revendications 1 à 7, dans lequel le circuit de détection physiologique (705 ; 805) comporte au moins un des éléments suivants :
un circuit de détection des signaux cardiaques ; un circuit de détection des bruits du coeur ;
un circuit de détection de la respiration ;
un circuit de détection de l'impédance cardiaque ; et un circuit de détection de l'activité physique.

9. Système de l'une quelconque des revendications 1 à 8,
dans lequel le circuit de détection physiologique (705 ; 805) est configuré pour produire des échantillons d'un signal physiologique détecté sous la forme d'au moins une partie des données physiologiques,
le système (700 ; 801, 803) comportant une interface utilisateur configurée pour recevoir une indication d'un segment spécifié du signal physiologique détecté pour lequel générer le rapport narratif.
